# EUROPÄISCHE PATENTSCHRIFT

(11) **EP 4 563 104 B1**
(45) Veröffentlichungstag und Bekanntmachung des Hinweises auf die Patenterteilung: **08.07.2026**
(21) Anmeldenummer: 24215826.9
(22) Anmeldetag: 27.11.2024
(51) Int. Cl.: A61B 17/70, A61B 17/28, A61B 17/29, B25B 7/08, B25B 7/14

(54) **MEDIZINISCHES BZW. CHIRURGISCHES INSTRUMENT ZUR ERGONOMISCHEN EINHANDBEDIENUNG**
MEDICAL OR SURGICAL INSTRUMENT FOR ERGONOMIC ONE-HAND OPERATION
INSTRUMENT MÉDICAL OU INSTRUMENT CHIRURGICAL POUR UNE COMMANDE ERGONOMIQUE À UNE SEULE MAIN

(30) Priorität: 28.11.2023 DE 102023133251
(43) Veröffentlichungstag der Anmeldung: 04.06.2025
(73) Patentinhaber: Aesculap AG, 78532 Tuttlingen (DE)
(72) Erfinder: LINKE, Ralph, 78234 Engen (DE); RICCI, Denis, 78573 Wurmlingen (DE)
(74) Vertreter: Winter, Brandl - Partnerschaft mbB

(56) Entgegenhaltungen:
- US-A1- 2003 009 168
- US-A1- 2006 166 535
- US-A1- 2008 195 129
- US-A1- 2009 299 141
- US-B1- 8 777 953

## Beschreibung

Die Offenbarung betrifft ein medizinisches bzw. chirurgisches Instrument mit: einer ersten Instrumentenbranche, die einen ersten proximalen Instrumentenbetätigungsabschnitt aufweist; einer zweiten Instrumentenbranche, die einen zweiten proximalen Instrumentenbetätigungsabschnitt aufweist; einem an der ersten Instrumentenbranche, vorzugsweise an dem ersten proximalen Instrumentenbetätigungsabschnitt, angebundenen Zahnstangenelement; und einer an der zweiten Instrumentenbrache, vorzugsweise an dem zweiten proximalen Instrumentenbetätigungsabschnitt, vorgesehenen Rastnase, mit welcher das Zahnstangenelement in verrastenden Eingriff bringbar ist.

### Stand der Technik

Pedikelschrauben dienen prinzipiell zur dorsalen Stabilisierung der Wirbelsäule bei Frakturen, Tumoren, Entzündungen, Deformitäten und degenerativ bedingten Instabilitäten mittels transpedikulärer Verschraubung. Dabei werden Pedikelschrauben in den Pedikeln jeweils benachbarter Wirbel platziert, worauf eine winkelstabile Verbindung zwischen den jeweils axial übereinander sich anordnenden Pedikelschrauben und einem axial sich erstreckenden Längsträger/ Stab geschaffen wird. Die Pedikelschrauben und Längsträger bilden dabei ein Wirbel-Stabilisierungssystem.

Hierfür hat eine Pedikelschraube in der Regel einen axialen, schaftartigen Außengewindeabschnitt, an den sich schraubenkopfseitig eine sogenannte Tulpe bzw. Aufnahmehülse anschließt. Diese bildet konstruktiv eine U-förmig geschlitzte/ getunnelte Aufnahmehülse mit Innengewinde, wobei die beiden sich radial gegenüberliegenden Längsschlitze jeweils einen Schlitzspalt vorbestimmter Spaltbreite definieren. In die parallel zueinander verlaufenden Längsschlitze ist der Längsträger/ Stab quer eingelegt, der mittels eines Verriegelungselements, zum Beispiel in Form einer Madenschraube, Gewindemutter oder Setscrew, die in das Innengewinde eingedreht ist, fixiert wird.

Bei der Operation eines Patienten werden üblicherweise zunächst die Pedikel der benachbarten Wirbel präpariert. Dabei wird nach einer Bestimmung eines Schraubeneintrittspunktes am Pedikel zunächst mit einem Ankörner der Pedikelkanal eröffnet. Anschließend wird mit einem Trokar der Schraubenkanal geschaffen. Mit einem Gewindeschneider kann das Gewinde für die Pedikelschrauben geschnitten werden. Alternativ können die Pedikelschrauben selbstschneidend sein. Danach werden die Schrauben in die Pedikel gedreht und der Stab wird positioniert. Der Stab kann mit einer Stabbiegezange konturiert und mit einer Stabhaltezange eingesetzt werden. Mit einer Stabandrückzange kann das anschließende Platzieren der Verriegelungselemente, insbesondere der Verriegelungsschrauben, erleichtert werden. Schließlich werden die Verriegelungsschrauben eingesetzt bzw. eingedreht. Vor einem abschließenden Verriegeln können sowohl Kompressions- als auch Distraktionsmanöver durchgeführt werden. Hierbei wird zunächst eine Verriegelungsschraube vollständig festgezogen, um einen fixen Ausgangspunkt für die Kompression bzw. Distraktion zu haben. Mithilfe eine Kompressionszange oder einer Distraktionszange kann dann ein gewünschtes Repositionsmanöver, insbesondere eine Kompression oder Distraktion durchgeführt werden. Dabei setzt die Kompressionszange bzw. die Distraktionszange an den benachbarten Schraubenköpfen an, um diese über einen Kompressions- bzw. Distraktionsvorgang zu einer gewünschten Position zu bewegen. Sobald die gewünschte Reposition erreicht ist, können die verbleibende(n) Verriegelungsschraube(n) vollständig angezogen werden.

Bei einer derartigen Operation werden somit eine Vielzahl von zangenartigen medizinischen bzw. chirurgischen Instrumenten, insbesondere Brancheninstrumenten, speziell Zangen eingesetzt. Dabei kann es wünschenswert sein, die Branchen dieser Instrumente auf eine bestimmte Position zueinander zu verrasten bzw. zu verriegeln, oder gegebenenfalls auch ein stufenweises Verstellen der Branchen zu realisieren. Beispielsweise wird bei einer Stabhaltezange, wenn die Branchen zueinander verrastet bzw. verriegelt sind, erreicht, dass der Stab beim Einsetzen nicht in unbeabsichtigter Weise verloren gehen bzw. in den Operationsbereich fallen kann. Bei einer Kompressionszange bzw. einer Distraktionszange kann in vorteilhafter Weise eine gewünschte Kompression bzw. Distraktion stufenweise eingestellt werden.

Grundsätzlich ist es bekannt, die Branchen eines zangenartigen medizinischen bzw. chirurgischen Instruments, insbesondere im Bereich deren Griffe bzw. Griffabschnitte, mit einer Zahnstange zu verrasten. Dabei sind Instrumente bekannt, in welchem ein dauerhafter Eingriff vorgesehen ist, was mit der Gefahr eines Verspannens einhergeht.

Auch ist es bekannt, eine Zahnstange an einer der Branchen schwenkbar zu lagern, wobei Zähne der Zahnstange mit einer an der anderen der Branchen vorgesehenen Rastnase verrasten können und auch wieder außer Eingriff gebracht werden können. Wenn der sich im Eingriff befindliche Zahn der Zahnstange aus der Rastnase herausbewegt wird, können die Branchen des zangenartigen medizinischen bzw. chirurgischen Instruments stufenweise verstellt werden, beispielsweise indem ein benachbarter Zahn in Eingriff mit der Rastnase gebracht wird. Alternativ kann ein Öffnen der Branchen ermöglicht werden, wenn die Zahnstange außer Eingriff mit der Rastnase gebracht ist.

Bei bekannten derartig verrastbaren bzw. verriegelbaren Brancheninstrumenten tritt häufig das Problem auf, dass das Außereingriffbringen zwischen der Zahnstange und der Rastnase unergonomisch ist, insbesondere keine Einhandbetätigung ermöglicht ist. Außerdem gelangen Finger des Anwenders häufig in Kontakt mit der Zahnstange, was dazu führt, dass ein Handschuh des Anwenders beschädigt wird oder sogar der Anwender sich mit der Zahnstange, die wie eine Säge wirkt, in den Finger schneidet.

US 2006/166535 A1 offenbart ein Brancheninstrument umfassend Griffabschnitte, die relativ zueinander schwenkbar und auseinander vorgespannt sind, und Armabschnitte, die relativ zueinander und zu den Griffabschnitten schwenkbar sind. Um das Brancheninstrument in einem zusammengedrückten Zustand zu halten, können eine Zahnstange und eine Sperrklinke vorgesehen sein.

US 2003/009168 A1 offenbart ein chirurgisches Instrument umfassend einen ersten Arm und einen zweiten Arm, wobei an einem proximalen Ende des ersten Arms eine Ratschenhalterung befestigt werden kann. Eine Ratsche ist schwenkbar mit der Ratschenhalterung verbunden und erstreckt sich vom ersten Arm zum zweiten Arm. Ein proximales Ende des zweiten Arms weist eine Spitze auf, an der ein Finger vorgesehen ist, der mit der Ratsche in Eingriff kommen kann. Ein Daumenlösehebel ist schwenkbar verbunden mit der Spitze des zweiten Arms. Der Daumenlösehebel weist einen Hebelbetätigungsabschnitt auf, mit dem der Finger aus Eingriff mit der Ratsche gebracht werden kann.

US 2009/299141 A1 offenbart ein laparoskopisches chirurgisches Instrument, das ergonomisch und anthropometrisch korrekt konfiguriert ist.

### Kurzbeschreibung der Erfindung

Vor diesem Hintergrund ist es Aufgabe der vorliegenden Erfindung, die voranstehend genannten Nachteile zu vermeiden bzw. zumindest zu mindern, und ein medizinisches bzw. chirurgisches Instrument bereitzustellen, dessen Branchen zueinander verrastet werden können, und bei welchem ein ergonomisches und gefahrenarmes Lösen der Verrastung ermöglicht wird.

Diese Aufgabe wird durch ein medizinisches bzw. chirurgisches Instrument nach Anspruch 1 gelöst. Vorteilhafte Ausführungsformen sind in den Unteransprüchen beansprucht und nachfolgend erläutert.

### Kurzbeschreibung der Offenbarung

Die Offenbarung betrifft ein medizinisches bzw. chirurgisches Instrument, insbesondere Brancheninstrument, mit: einer ersten Instrumentenbranche, die einen ersten proximalen Instrumentenbetätigungsabschnitt aufweist; einer zweiten Instrumentenbranche, die einen zweiten proximalen Instrumentenbetätigungsabschnitt aufweist; einem an der ersten Instrumentenbranche, vorzugsweise an dem ersten proximalen Instrumentenbetätigungsabschnitt, angebundenen Zahnstangenelement; einer an der zweiten Instrumentenbrache, vorzugsweise an dem zweiten proximalen Instrumentenbetätigungsabschnitt, vorgesehenen Rastnase, mit welcher das Zahnstangenelement in verrastenden Eingriff bringbar ist; und einen, vorzugsweise ergonomisch betätigbaren und eine Einhand-Betätigung ermöglichenden, Betätigungshebel, welcher vorgesehen und eingerichtet ist, bei Betätigung desselben das Zahnstangenelement aus dem verrastenden Eingriff mit der Rastnase zu bringen. Das offenbarungsgemäße medizinisches bzw. chirurgische Instrument ist bevorzugt scherenartig oder zangenartig oder pinzettenartig ausgebildet ist, ist also insbesondere eine Schere oder eine Zange oder eine Pinzette. Besonders bevorzugt ist das medizinische bzw. chirurgische Instrument eine Zange, insbesondere eine Stabhaltezange oder eine Kompressionszange oder eine Distraktionszange.

Das medizinische bzw. chirurgische Instrument weist offenbarungsgemäß zwei Instrumentenbranchen auf, die vorzugsweise neben den proximalen Instrumentenbetätigungsabschnitten auch jeweils distale Eingriffsabschnitte, einen ersten distalen Eingriffsabschnitt und einen zweiten distalen Eingriffsabschnitt, aufweisen. Es ist zu verstehen, dass die beiden Instrumentenbranchen bevorzugt einteilig ausgebildet sind, das heißt, dass der erste proximale Instrumentenbetätigungsabschnitt und der erste distale Eingriffsabschnitt Abschnitte desselben Bauteils sind, und dass der zweite proximale Instrumentenbetätigungsabschnitt und der zweite distale Eingriffsabschnitt Abschnitte desselben Bauteils sind. Alternativ kann es jedoch auch vorgesehen sein, dass der erste proximale Instrumentenbetätigungsabschnitt an einem anderen Bauteil als der erste distale Eingriffsabschnitt vorgesehen ist und/oder dass der zweite proximale Instrumentenbetätigungsabschnitt an einem anderen Bauteil als der zweite distale Eingriffsabschnitt vorgesehen ist, wobei die beiden Bauteile beispielsweise über ein Gelenk miteinander verbunden sein können. In beiden Fällen ist es jedoch bevorzugt, dass eine Betätigung der beiden proximalen Instrumentenbetätigungsabschnitte zu einem Ineingriffbringen oder Außereingriffbringen der distalen Eingriffsabschnitte führt. In vorteilhafter Weise sind die erste Instrumentenbranche und die zweite Instrumentenbrache, das heißt die zwei Instrumentenbranchen gegeneinander/ relativ zueinander (ver-)schwenkbar gekoppelt. Die erste Instrumentenbranche und die zweite Instrumentenbrache können über eine Blattfederanordnung gegeneinander vorgespannt sein. Im Falle einer Scheren-/ Zangenbauweise kann das medizinische bzw. chirurgische Instrument ein Scharniergelenk haben, welches zwischen den proximalen Instrumentenbetätigungsabschnitten und den distalen Eingriffsabschnitten vorgesehen bzw. angeordnet ist. Im Falle einer Pinzettenbauweise können die zwei Instrumentenbranchen an ihren proximalen Enden über ein Federscharnier, insbesondere einen Blattfederabschnitt, miteinander gekoppelt sein.

Die distalen Eingriffsabschnitte können Halteabschnitte oder Greifabschnitte oder Verformungsabschnitte, insbesondere Kompressionsabschnitte oder Distraktionsabschnitte, oder Schneide- bzw. Trennabschnitte oder Ähnliches sein. Sie können also bevorzugt zum Halten oder zum Greifen oder zum Verformen, insbesondere zur Kompression/ zum Komprimieren oder zur Distraktion/ zum Distrahieren, insbesondere eines Implantats, etwa einer Pedikelschraube oder eines Stabs, vorbereitet und ausgebildet sein. Alternativ oder zusätzlich können sie zum Durchführen/ Ausführen eines ähnlichen Manövers, etwa eines Schneidens oder Trennens vorbereitet und ausgebildet sein. Allgemeiner ausgedrückt sind die distalen Eingriffsabschnitt vorgesehen und ausgebildet, in ein Implantat, etwa in eine Pedikelschraube oder einen Stab, einzugreifen.

Die proximalen Instrumentenbetätigungsabschnitte sind grundsätzlich vorgesehen und ausgebildet, um von einem Anwender insbesondere einhändig gegriffen und betätigt zu werden. Die proximalen Instrumentenbetätigungsabschnitte können, insbesondere ergonomische, Branchengriffflächen aufweisen, das heißt der erste proximale Instrumentenbetätigungsabschnitt kann eine erste Branchengrifffläche aufweisen und der zweite proximale Instrumentenbetätigungsabschnitt kann eine zweite Branchengrifffläche aufweisen. Die proximalen Instrumentenbetätigungsabschnitte können ergonomische Riffelungen oder Rillen oder Rippen aufweisen, welche dem Anwender ein taktiles Feedback geben, so dass der Anwender weiß, dass er das entsprechende medizinische bzw. chirurgische Instrument an der dafür vorgesehenen Stelle hält bzw. greift. Die proximalen Instrumentenbetätigungsabschnitte können ferner jeweils distale Vorsprünge aufweisen, welche einen von dem Anwender zu greifenden bzw. haltenden Bereich begrenzen.

Die proximalen Instrumentenbetätigungsabschnitte sind insbesondere vorgesehen, um von einer Hand eines Anwenders umgriffen zu werden, insbesondere derart, dass Finger der Hand an dem ersten proximalen Instrumentenbetätigungsabschnitt, insbesondere an der ersten Branchengrifffläche anliegen, und dass ein Handballen oder ein Mittelhandabschnitt der Hand an dem zweiten proximalen Instrumentenbetätigungsabschnitt, insbesondere an der zweiten Branchengrifffläche anliegt. Wenn offenbarungsgemäß von einem ergonomisch betätigbaren und eine Einhand-Betätigung ermöglichenden Betätigungshebel die Rede ist, ist darunter insbesondere ein Betätigungshebel zu verstehen, welcher in diesem Halte- bzw. Greifzustand des Anwenders von dem Anwender mit seinem Daumen erreichbar ist, der also insbesondere an oder nahe dem zweiten proximalen Instrumentenbetätigungsabschnitt (insbesondere nahe dessen proximalem Ende) der zweiten Instrumentenbranche vorgesehen und angeordnet ist, wobei insbesondere ein Betätigungsabschnitt oder eine Betätigungsfläche des Betätigungshebels von dem zweiten proximalen Instrumentenbetätigungsabschnitt der zweiten Instrumentenbranche (geeignet) für eine ergonomische Betätigung mit dem Daumen der Hand beabstandet ist.

Das vorgesehene Zahnstangenelement, welches insbesondere eine Zahnstange ist, ist in vorteilhafter Weise an der ersten Instrumentenbranche schwenkbar gelagert bzw. ist in Bezug auf die erste Instrumentenbranche verschwenkbar. Besonders bevorzugt ist das Zahnstangenelement an dem ersten proximalen Instrumentenbetätigungsabschnitt, insbesondere an einem proximalen Ende des ersten proximalen Instrumentenbetätigungsabschnitts, schwenkbar gelagert bzw. ist in Bezug auf den ersten proximalen Instrumentenbetätigungsabschnitt verschwenkbar. Somit kann das Zahnstangenelement in vorteilhafter Weise über eine Schwenkbewegung mit der Rastnase in Eingriff und außer Eingriff gebracht werden.

Das Zahnstangenelement kann eine Vielzahl von Zähnen aufweisen, welche jeweils vorgesehen und eingerichtet sind, mit der an der zweiten Instrumentenbranche vorgesehenen Rastnase in verrastenden Eingriff gebracht zu werden. Dabei ist insbesondere eine Öffnungs- bzw. Schließweite der zwei Instrumentenbranchen, insbesondere der distalen Eingriffsabschnitte der zwei Instrumentenbranchen, über den sich mit der Rastnase in Eingriff befindlichen Zahn der Vielzahl von Zähnen einstellbar. Bevorzugt kann somit, wenn die Öffnungs- bzw. Schließweite der zwei Instrumentenbrachen verändert insbesondere verstellt werden soll, das Zahnstangenelement insbesondere mittels dem vorgesehenen Betätigungshebel mit der Rastnase außer Eingriff gebracht werden, die Öffnungs- bzw. Schließweite kann im Anschluss verändert werden, um bei der neu einzustellenden Öffnungs- bzw. Schließweite die beiden Instrumentenbranchen wieder zu verrasten, indem ein anderer Zahn der Vielzahl von Zähnen (bzw. ein anderer Zwischenraum zwischen zwei Zähnen) mit der Rastnase von dem Anwender verrastet wird, insbesondere das Zahnstangenelement bei der neu einzustellenden Öffnungs- bzw. Schließweite wieder in bzw. gegen die Rastnase gedrückt wird. Die Rastnase ist somit bevorzugt an dem zweiten proximalen Betätigungsabschnitt der zweiten Instrumentenbranche, insbesondere an einem proximalen Ende des zweiten proximalen Betätigungsabschnitts, vorgesehen bzw. angeordnet. Der Betätigungshebel und die Rastnase sind bevorzugt als separate Bauteile ausgebildet. Mit anderen Worten ist die Rastnase bevorzugt kein integraler Bestandteil des Betätigungshebels. Vorzugsweise sind der Betätigungshebel und das Zahnstangenelement separat ausgebildet, d.h., das Zahnstangenelement ist kein integraler Bestandteil des Betätigungshebels. In einer bevorzugten Ausführungsform sind der Betätigungshebel, die Rastnase und das Zahnstangenelement jeweils als separate Teile ausgebildet.

Der Betätigungshebel, welcher auch als ein Entsperrhebel bezeichnet werden kann, ist bevorzugt schwenkbar an der zweiten Instrumentenbranche, insbesondere an dem zweiten proximalen Instrumentenbetätigungsabschnitt, bevorzugt an einem proximalen Ende desselben, besonders bevorzugt nahe der Rastnase, gelagert.

Der Betätigungshebel weist einen Hebelbetätigungsabschnitt mit einer Betätigungsfläche auf, welche bzw. welcher vorgesehen ist, von einem Anwender betätigt zu werden.

Ferner kann der Betätigungshebel einen Hebellagerabschnitt aufweisen, über welchen der Betätigungshebel schwenkbar/ drehbar an der zweiten Instrumentenbranche gelagert ist. Außerdem kann der Betätigungshebel einen Hebeleingriffsabschnitt aufweisen, welcher vorgesehen und eingerichtet ist, mit dem Zahnstangenelement, insbesondere mit einem Zahn oder Zähnen des Zahnstangenelements, in Kontakt bzw. Eingriff zu sein oder gebracht zu werden, um das Zahnstangenelement aus der Rastnase zu hebeln, insbesondere bei einer Dreh- bzw. Schwenkbewegung des Betätigungshebels.

Der Betätigungshebel kann eine erste Nichteingriffsstellung und eine zweite Eingriffsstellung haben. Der Betätigungshebel kann zwischen der ersten Nichteingriffsstellung und der zweiten Eingriffsstellung verschwenkbar bzw. verdrehbar sein. In der ersten Nichteingriffsstellung liegt insbesondere der verrastende Eingriff zwischen dem Zahnstangenelement und der Rastnase vor, und in der zweiten Eingriffsstellung liegt insbesondere der verrastende Eingriff zwischen dem Zahnstangenelement und der Rastnase nicht vor, ist das Zahnstangenelement somit insbesondere aus der Rastnase (heraus-)gehebelt. Der Betätigungshebel kann in eine der Stellungen, also entweder in die erste Nichteingriffsstellung oder in die zweite Eingriffsstellung vorgespannt sein, insbesondere über ein Federelement. Beispielsweise kann der Betätigungshebel in die erste Nichteingriffsstellung vorgespannt sein, wodurch ein versehentliches Außereingriffbringen von Zahnstangenelement und Rastnase besser vermieden werden kann.

Wenn offenbarungsgemäß von einem ergonomisch betätigbaren und eine Einhand-Betätigung ermöglichenden Betätigungshebel die Rede ist, ist darunter insbesondere ein Betätigungshebel zu verstehen, welcher in dem voranstehend angesprochenen Halte- bzw. Greifzustand des Anwenders von dem Anwender mit seinem Daumen erreichbar ist, der also insbesondere an oder nahe dem zweiten proximalen Instrumentenbetätigungsabschnitt der zweiten Instrumentenbranche vorgesehen und angeordnet ist, wobei insbesondere der (Hebel-)Betätigungsabschnitt oder die Betätigungsfläche des Betätigungshebels von dem zweiten proximalen Instrumentenbetätigungsabschnitt der zweiten Instrumentenbranche (geeignet) für eine ergonomische Betätigung mit dem Daumen der Hand beabstandet ist, und wobei insbesondere zusätzlich der (Hebel-)Betätigungsabschnitt bzw. zumindest die Betätigungsfläche des (Hebel-)Betätigungsabschnitts von dem Zahnstangenelement (geeignet) für eine ergonomische Betätigung mit dem Daumen der Hand beabstandet ist. Allgemeiner gesagt ist der (Hebel-)Betätigungsabschnitt und insbesondere die Betätigungsfläche des (Hebel-)Betätigungsabschnitts insbesondere zumindest zum Teil von sowohl der zweiten Instrumentenbranche/ dem zweiten proximalen Instrumentenbetätigungsabschnitt als auch von dem Zahnstangenelement beabstandet, insbesondere von einem Raum, der durch den zweiten proximalen Instrumentenbetätigungsabschnitt und die Zahnstange aufgespannt bzw. definiert ist, seitlich versetzt bzw. beabstandet, speziell von einer den Raum seitlich begrenzenden Randebene beabstandet und versetzt und somit außerhalb des Raums angeordnet.

In anderen Worten kann man sagen, dass die erste Instrumentenbranche, die zweite Instrumentenbranche und das Zahnstangenelement einen (medialen) Raum aufspannen bzw. definieren, insbesondere der erste proximale Instrumentenbetätigungsabschnitt, der zweite proximale Instrumentenbetätigungsabschnitt und das Zahnstangenelement den (medialen) Raum aufspannen bzw. definieren, und der Hebelbetätigungsabschnitt, insbesondere die Betätigungsfläche des Hebelbetätigungsabschnitts, des Betätigungshebels (zumindest teilweise) außerhalb des Raums angeordnet ist. Der (mediale) Raum ist somit bevorzugt ein Raum, welcher durch den (proximalen) Griffbereich und den (distalen) Schließ- bzw. Eingriffsbereich der (Instrumenten-) Branchen, sowie des Zahnstangenelements/ der Zahnstange umfasst ist. Der Betätigungshebel/ Entsperrhebel hat bevorzugt eine Grifffläche/ Betätigungsfläche außerhalb dieses medialen Raums.

In vorteilhafter Weise weist das Zahnstangenelement eine die Zähne ausbildende bzw. aufweisende Zahneingriffsfläche, vorzugsweise ist die Zahneingriffsfläche durchgehend mit Zähnen belegt, eine der Zahneingriffsfläche gegenüberliegende Nichteingriffsfläche und zwei Seitenflächen, eine erste Seitenfläche und eine zweite Seitenfläche auf, welche die Zahneingriffsfläche und die Nichteingriffsfläche verbinden, wobei der Hebelbetätigungsabschnitt zumindest teilweise, insbesondere die Betätigungsfläche des Hebelbetätigungsabschnitts bevorzugt vollständig, von der ersten Seitenfläche in einer Richtung weg von der Zahneingriffsfläche beabstandet ist und/oder sich der Hebelbetätigungsabschnitt in Bezug auf die erste Seitenfläche von dieser wegerstreckt, um den Hebelbetätigungsabschnitt und insbesondere die Betätigungsfläche des Hebelbetätigungsabschnitts von den Zähnen des Zahnstangenelements zu beabstanden. Wenn die Betätigungsfläche des Hebelbetätigungsabschnitts vollständig, das heißt die gesamte Betätigungsfläche, von der vorstehend angesprochenen ersten Seitenfläche in einer Richtung weg von der Zahneingriffsfläche beabstandet ist, wird erreicht, dass ein Finger des Anwenders, insbesondere der Daumen, nicht versehentlich die Zähne des Zahnstangenelements berührt, was mit der Gefahr eines Einschneidens in einen Handschuh bzw. im schlimmsten Fall sogar mit einer Schnittwunde an dem Finger/ dem Daumen des Anwenders einhergehen würde. Es ist zu verstehen, dass die erste Seitenfläche, die Nichteingriffsfläche und die zweite Seitenfläche einen Knick ausbilden können bzw. zueinander angewinkelt, insbesondere (näherungsweise) in einem rechten Winkel, sein können oder gerundete Übergänge aufweisen können oder insgesamt gerundet ineinander übergehen können, um einen das Zahnstangenelement ausbildenden Körper auszubilden.

Der Hebelbetätigungsabschnitt ist ein abgewinkelter Abschnitt oder ein gekrümmter Abschnitt oder ein gewölbter Abschnitt. Der Betätigungshebel/ Entsperrhebel kann somit als abgewinkelter/ gekrümmter/ gewölbter Betätigungshebel/ Entsperrhebel bezeichnet werden. Der Hebelbetätigungsabschnitt ist in Bezug auf die Zähne der Zahnstange in distale Richtung weggekrümmt bzw. weggewölbt, um den Hebelbetätigungsabschnitt, insbesondere dessen Betätigungsfläche in zwei, vorzugsweise aufeinander senkrecht stehenden, Raumrichtungen von den Zähnen des Zahnstangenelements zu beabstanden. Der Hebelbetätigungsabschnitt ist ein gekrümmter oder gewölbter Abschnitt, welcher insbesondere in Bezug auf die Zähne der Zahnstange weggekrümmt bzw. weggewölbt ist,

In vorteilhafter Weise weist der Hebelbetätigungsabschnitt eine abgewinkelte oder gewölbte oder gekrümmte Grifffläche/ Betätigungsfläche auf, welche in Bezug auf den bzw. relativ zu dem (medialen) Raum abgewinkelt oder gewölbt oder gekrümmt ist.

Es ist von Vorteil, wenn sich der Hebelbetätigungsabschnitt des Betätigungshebels im Wesentlichen in einer Richtung von den Instrumentenbranchen und dem Zahnstangenelement weg erstreckt, welche näherungsweise parallel zu einer Schwenkachse ist, um welche die beiden Instrumentenbranchen zueinander verschwenkbar sind, wobei der Hebelbetätigungsabschnitt im Bereich seiner Betätigungsfläche zusätzlich gekrümmt ausgebildet ist, insbesondere von den Zähnen des Zahnstangenelements weggekrümmt wird, um eine ergonomische Betätigungsfläche bereitzustellen und die Betätigungsfläche von den Zähnen des Zahnstangenelements weiter zu beabstanden.

Es kann von Vorteil sein, wenn sich der Hebelbetätigungsabschnitt im Wesentlichen in einer Richtung von den Branchen und dem Zahnstangenelement weg erstreckt, welche näherungsweise parallel zu einer Schwenkachse ist, um welche der Betätigungshebel in Bezug auf die zweite Instrumentenbranche verschwenkbar ist, wobei der Hebelbetätigungsabschnitt im Bereich seiner Betätigungsfläche zusätzlich gekrümmt ausgebildet ist, insbesondere von den Zähnen des Zahnstangenelements weggekrümmt wird, um eine ergonomische Betätigungsfläche bereitzustellen und die Betätigungsfläche von den Zähnen des Zahnstangenelements weiter zu beabstanden.

Allgemeiner ausgedrückt kann die Betätigungsfläche des Hebelbetätigungsabschnitts ergonomisch gekrümmt oder gewölbt sein, um somit für einen Finger, insbesondere einen Daumen, des Anwenders eine ergonomisch angenehme Betätigungsfläche bereitzustellen. Die Betätigungsfläche ist somit in anderen Worten bevorzugt eine gekrümmte oder gewölbte Fläche und somit keine ebene Fläche.

Die Betätigungsfläche des Hebelbetätigungsabschnitts kann gerippt oder gewellt oder gerillt ausgebildet sein, insbesondere Rippen oder Wellen oder Rillen aufweisen, für eine ergonomische Betätigung der Betätigungsfläche des Hebelbetätigungsabschnitts. Die Betätigungsfläche des Hebelbetätigungsabschnitts kann in ihrer Größe an eine Fingerspitze eines Daumens angepasst sein, insbesondere eine Fläche von 0,5 bis 3 cm², bevorzugt 0,5 bis 1,5 cm² aufweisen.

Der Lagerabschnitt des Betätigungshebels kann Zapfen/ zapfenförmige Vorsprünge aufweisen, welche vorgesehen und eingerichtet sind, um in an der zweiten Instrumentenbranche vorgesehene Ausnehmungen eingebracht zu werden, für eine schwenkbare Lagerung des Betätigungshebels an der zweiten Instrumentenbranche.

Der Hebeleingriffsabschnitt des Betätigungshebels kann ballenartig/ nach Art eines Ballens oder kugelartig/ nach Art einer Kugel ausgebildet sein, insbesondere gerundet/ abgerundet sein, speziell in einem Bereich bzw. Abschnitt welcher vorgesehen ist, in Kontakt bzw. Eingriff mit dem Zahnstangenelement, insbesondere den Zähnen des Zahnstangenelements, zu kommen. Wenn dieser Bereich bzw. Abschnitt gerundet bzw. abgerundet und somit nicht eckig und nicht kantig ist, wird eine Gefahr reduziert, dass sich der Hebeleingriffsabschnitt mit dem Zahnstangenelement/ den Zähnen des Zahnstangenelements verspannt bzw. unlösbar oder schwer lösbar in Eingriff gelangt.

Vorteilhaft ist, dass der Hebelbetätigungsabschnitt an einem Ende ein knaufförmiges oder kugelförmiges oder ovalförmiges Gebilde aufweist. Insbesondere kann eine Oberfläche des knaufförmigen oder kugelförmigen oder ovalförmigen Gebildes die Betätigungsfläche des Hebelbetätigungsabschnitts bilden. In einer bevorzugten Ausführungsform ist die Betätigungsfläche des Hebelbetätigungsabschnitts zusätzlich gerippt und/oder gewellt und/oder gerillt ausgebildet und/oder mit Noppen versehen.

### Kurzbeschreibung der Figuren

Die Offenbarung wird nachfolgend anhand von Figuren weiter erläutert. Es zeigen:
- Fig. 1: eine Vorderansicht eines offenbarungsgemäßen medizinischen bzw. chirurgischen Instruments in Form einer Distraktionszange;
- Fig. 2: eine Unteransicht der Distraktionszange von Fig. 1;
- Fig. 3: eine Vorderansicht eines Zahnstangenelements der Distraktionszange gemäß Fig. 1;
- Fig. 4: eine Draufsicht des Zahnstangenelements gemäß Fig. 3;
- Fig. 5: eine Vorderansicht eines Betätigungshebels der Distraktionszange gemäß Fig. 1;
- Fig. 6: eine Draufsicht des Betätigungshebels gemäß Fig. 5;
- Fig. 7: einen Schnitt durch einen Hebelbetätigungsabschnitt des Betätigungshebels gemäß den Fign. 5 und 6;
- Fig. 8: eine Vorderansicht eines offenbarungsgemäßen medizinischen bzw. chirurgischen Instruments in Form einer Kompressionszange;
- Fig. 9: eine Unteransicht der Kompressionszange von Fig. 8;
- Fig. 10: eine Vorderansicht eines offenbarungsgemäßen medizinischen bzw. chirurgischen Instruments in Form einer Stabhaltezange; und
- Fig. 11: eine Unteransicht der Stabhaltezange von Fig. 10.

Die Figuren sind lediglich schematischer Natur und dienen ausschließlich dem besseren Verständnis der Offenbarung. Gleiche Elemente sind mit denselben Bezugszeichen versehen. Die Merkmale der einzelnen Ausführungsformen können untereinander ausgetauscht und kombiniert werden.

### Detaillierte Beschreibung der bevorzugten Ausführungsformen

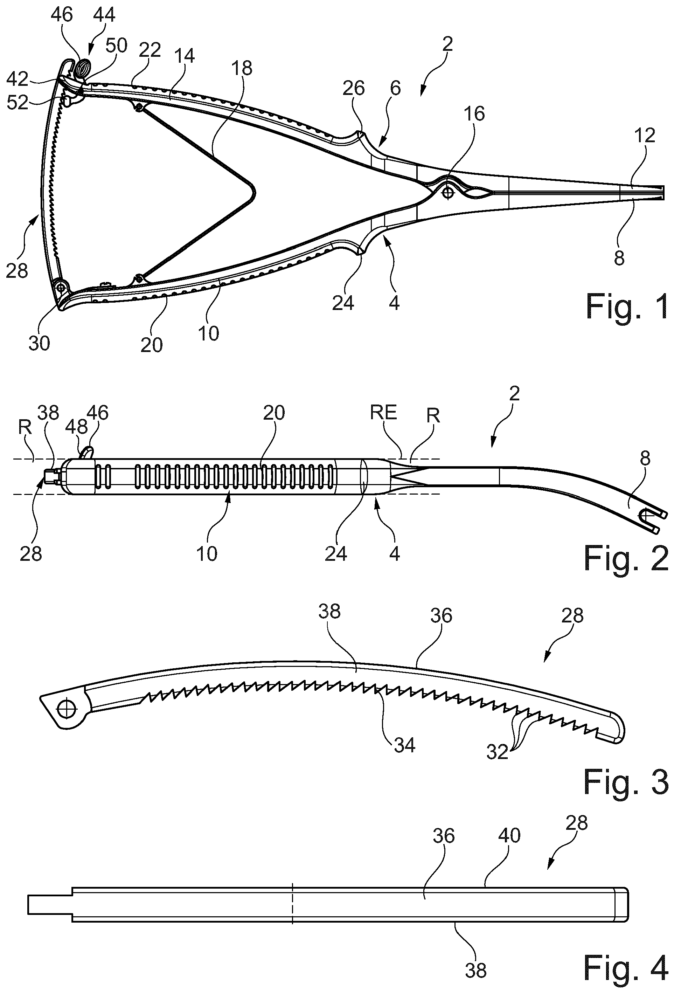

Fig. 1 zeigt eine Vorderansicht und Fig. 2 zeigt eine Unteransicht eines erfindungsgemäßen medizinischen bzw. chirurgischen Instruments 2 in Form einer Distraktionszange, mittels welcher ein Distraktionsmanöver an Schraubenköpfen von benachbarten Pedikelschrauben während eines Operationsvorgangs durchgeführt werden kann, um die Schraubenköpfe zu einer gewünschten Position zu bewegen und somit zu repositionieren. Das medizinische bzw. chirurgische Instrument 2 ist somit ein zangenartiges Brancheninstrument mit einer ersten Instrumentenbranche 4 und einer zweiten Instrumentenbranche 6. Die erste Instrumentenbranche 4 weist einen ersten distalen Eingriffsabschnitt 8 und einen ersten proximalen Instrumentenbetätigungsabschnitt 10 auf. Die zweite Instrumentenbranche 6 weist einen zweiten distalen Eingriffsabschnitt 12 und einen zweiten proximalen Betätigungsabschnitt 14 auf. Das medizinische bzw. chirurgische Instrument 2 weist ein Scharniergelenk 16 auf, welches zwischen den proximalen Instrumentenbetätigungsabschnitten 10, 14 und den distalen Eingriffsabschnitten 8, 12 vorgesehen bzw. angeordnet ist. Die erste Instrumentenbranche 4 und die zweite Instrumentenbranche 6 sind an dem Scharniergelenk 16 relativ zueinander verschwenkbar gekoppelt, und zwar derart, dass ein Aufeinander-Zu-Bewegen der proximalen Instrumentenbetätigungsabschnitte 10, 14 ein Auseinander-Bewegen der distalen Eingriffsabschnitte 8, 12, welche gemäß der Ausführungsform der Fig. 1 auch als Distraktionsabschnitte bezeichnet werden können, bewirkt, wodurch das angesprochene Distraktionsmanöver durchführbar ist. Dies wird insbesondere dadurch erreicht, dass die beiden Instrumentenbranchen 4, 6 an dem Scharniergelenk 16 sich nicht überkreuzen. Die erste Instrumentenbranche 4 und die zweite Instrumentenbrache 6 sind über eine Blattfederanordnung 18 gegeneinander vorgespannt, insbesondere hin zu einer geschlossenen Position der distalen Eingriffsabschnitte 8, 12.

Die proximalen Instrumentenbetätigungsabschnitte 10, 14 weisen erste und zweite Branchengriffflächen 20, 22 auf, welche in distaler Richtung von ersten und zweiten (distalen) Vorsprüngen 24, 26 begrenzt sind. Ein Anwender greift die proximalen Instrumentenbetätigungsabschnitte 10, 14 üblicherweise derart, dass Finger seiner Hand an der ersten Branchengrifffläche 20 anliegen, und dass ein Handballen oder ein Mittelhandabschnitt seiner Hand an der zweiten Branchengrifffläche 22 anliegt. In diesem Halte- bzw. Greifzustand des medizinischen bzw. chirurgischen Instruments 2 durch den Anwender befindet sich der Daumen des Anwenders grundsätzlich in einem proximalen Endbereich des zweiten proximalen Betätigungsabschnitts 14.

Das medizinische bzw. chirurgische Instrument 2 weist ferner ein Zahnstangenelement 28 in Form einer Zahnstange auf, welches im Nachfolgenden auch unter Bezugnahme auf die Fign. 3 und 4 beschrieben wird. Das Zahnstangenelement 28 ist an die erste Instrumentenbranche 4 angebunden und dabei an einem proximalen Ende des ersten proximalen Instrumentenbetätigungsabschnitts 10 über ein Gelenk 30 schwenkbar gelagert, so dass das Zahnstangenelement 28 relativ zu der ersten Instrumentenbranche 4 verschwenkbar ist. Das Zahnstangenelement 28 weist eine Vielzahl von Zähnen 32 auf, wobei eine die Zähne 32 aufweisende Fläche des Zahnstangenelements 28 eine Zahneingriffsfläche 34 bildet. Dieser Zahneingriffsfläche 34 liegt eine Nichteingriffsfläche 36 gegenüber. Eine erste Seitenfläche 38 und eine zweite Seitenfläche 40 verbinden die Zahneingriffsfläche 34 mit der Nichteingriffsfläche 36. In dem gezeigten Ausführungsbeispiel stehen die Flächen 34, 36, 38 und 40 im Wesentlichen aufeinander senkrecht und begrenzen einen das Zahnstangenelement 28 ausbildenden Körper.

An der zweiten Instrumentenbrache 6, insbesondere an einem proximalen Ende des zweiten proximalen Betätigungsabschnitts 14, ist eine Rastnase 42 vorgesehen, mit welcher das Zahnstangenelement 28, insbesondere dessen Zähne 32 in verrastenden Eingriff bringbar ist. Die Rastnase 42 ist entsprechend komplementär zu den Zähnen 32 ausgebildet, insbesondere derart, dass, wenn das Zahnstangenelement 28 mit seinen Zähnen 32 in bzw. gegen die Rastnase 42 gedrückt ist und somit eine Verrastung vorliegt, keine Relativbewegung der beiden Instrumentenbranchen 4, 6 möglich ist. Das Zahnstangenelement 28 kann über eine Schwenkbewegung mit der Rastnase 42 in Eingriff und außer Eingriff gebracht werden. Eine Öffnungs- bzw. Schließweite der zwei Instrumentenbranchen 4, 6, insbesondere der distalen Eingriffsabschnitte 8, 12 der zwei Instrumentenbranchen 4, 6, ist über den sich mit der Rastnase 42 in Eingriff befindlichen Zahn 32 der Vielzahl von Zähnen 32 (bzw. den entsprechenden Zwischenraum zwischen zwei Zähnen 32) einstellbar.

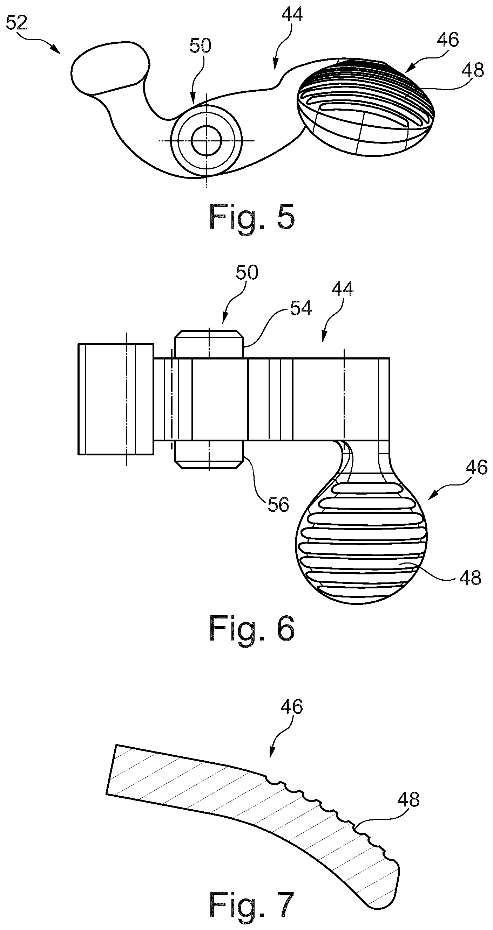

Das medizinische bzw. chirurgische Instrument 2 weist ferner einen ergonomisch betätigbaren und eine Einhand-Betätigung ermöglichenden Betätigungshebel 44 auf, welcher im Nachfolgenden auch unter Bezugnahme auf die Fign. 5, 6 und 7 beschrieben wird. Der Betätigungshebel 44 ist schwenkbar an der zweiten Instrumentenbranche 6, insbesondere an bzw. nahe einem proximalen Ende des zweiten proximalen Instrumentenbetätigungsabschnitts 14 und somit nahe der Rastnase 42 gelagert. Der Betätigungshebel 44 weist einen Hebelbetätigungsabschnitt 46 mit einer Betätigungsfläche 48 auf, welche vorgesehen ist, von einem Anwender betätigt zu werden. Der Betätigungshebel 44 weist ferner einen Hebellagerabschnitt 50 auf, über welchen der Betätigungshebel 44 schwenkbar bzw. drehbar an der zweiten Instrumentenbranche 6 gelagert ist. Außerdem weist der Betätigungshebel 44 einen Hebeleingriffsabschnitt 52 auf, welcher vorgesehen und eingerichtet ist, mit dem Zahnstangenelement 28, insbesondere mit einem Zahn 32 oder Zähnen 32 des Zahnstangenelements 28, in Kontakt bzw. Eingriff zu sein oder gebracht zu werden, um das Zahnstangenelement 28 aus der Rastnase 42 zu hebeln, insbesondere bei einer Dreh- bzw. Schwenkbewegung des Betätigungshebels 44. In Fig. 1 ist der Betätigungshebel 44 in einer ersten Nichteingriffsstellung gezeigt, in welcher der verrastende Eingriff zwischen dem Zahnstangenelement 28 und der Rastnase 42 vorliegt. Wird der Betätigungshebel 44 ausgehend von der in Fig. 1 gezeigten Stellung im Uhrzeigersinn verschwenkt, wird das Zahnstangenelement 28 durch den Hebeleingriffsabschnitt 52 des Betätigungshebels 44 aus der Rastnase 42 herausgehebelt und der Betätigungshebel 44 befindet sich in einer zweiten Eingriffsstellung. Wenn das Zahnstangenelement 28 außer Eingriff mit der Rastnase 42 gebracht ist, kann eine Öffnungs- bzw. Schließweite der zwei Instrumentenbrachen 4, 6 verstellt werden. Nach der Verstellung der Öffnungs- bzw. Schließweite der zwei Instrumentenbrachen 4, 6 kann das Zahnstangenelement 28 mit der Rastnase 42 auf einfache Weise wieder verrastet werden, indem ein Anwender auf das Zahnstangenelement 28, insbesondere auf die Nichteingriffsfläche 36 des Zahnstangenelements 28 drückt. Der Betätigungshebel 44 kann in die Nichteingriffsstellung über ein (nicht gezeigtes) Federelement vorgespannt sein, um ein unbeabsichtigtes Außereingriffbringen zwischen dem Zahnstangenelement 28 und der Rastnase 42 besser vermeiden zu können.

Erfindungsgemäß ermöglicht der Betätigungshebel 44 aufgrund seiner Ausbildung und Anordnung an dem medizinischen bzw. chirurgischen Instrument 2 eine ergonomische Einhand-Betätigung, insbesondere durch den Daumen des Anwenders, und zwar dem Daumen der Hand des Anwenders, welche auch die proximalen Betätigungsabschnitte 10, 14 umgreift. Insbesondere ist der Hebelbetätigungsabschnitt 46 und dabei ganz besonders die Betätigungsfläche 48 des Hebelbetätigungsabschnitts 46 (zumindest abschnittsweise) von sowohl dem zweiten proximalen Betätigungsabschnitt 14 der zweiten Instrumentenbranche 6 als auch von dem Zahnstangenelement 28 beabstandet, und ist zusätzlich der Hebelbetätigungsabschnitt 46 und dabei ganz besonders die Betätigungsfläche 48 des Hebelbetätigungsabschnitts 46 auch noch von dem Zahnstangenelement 28 und dem proximalen Ende des zweiten proximalen Betätigungsabschnitts 14 weggekrümmt bzw. weggewölbt.

Wenn man mit Bezugnahme auf die Unteransicht in Fig. 2 beispielsweise einen (medialen) Raum R betrachtet, welcher von den proximalen Betätigungsabschnitten 10, 14 aufgespannt bzw. definiert wird, das heißt sich aus der in Fig. 2 gezeigten Ebene heraus- und hineinerstreckt, und in welchem sich somit das Zahnstangenelement 28 vollständig befindet, gilt, dass der Hebelbetätigungsabschnitt 46 des Betätigungshebels 44 und insbesondere die Betätigungsfläche 48 des Hebelbetätigungsabschnitts 46 zumindest teilweise außerhalb des Raums R angeordnet sind. Der Hebelbetätigungsabschnitt 46 bzw. dessen Betätigungsfläche 48 stehen somit bezüglich dieses Raums R vor und sind von diesem zumindest teilweise seitlich versetzt bzw. beabstandet, insbesondere von einer den Raum seitlich begrenzenden Randebene RE. Wenn in Fig. 2 das Zahnstangenelement 28 und die Betätigungsfläche 48 des Hebelbetätigungsabschnitts 46 betrachtet werden, verhält es sich sogar so, dass die Betätigungsfläche 48 des Hebelbetätigungsabschnitts 46 vollständig (das heißt die gesamte Betätigungsfläche 48) von der ersten Seitenfläche 38 in einer Richtung weg von der Zahneingriffsfläche 34 beabstandet ist und sich der Hebelbetätigungsabschnitt 46 in Bezug auf die erste Seitenfläche 38 von dieser wegerstreckt, um den Hebelbetätigungsabschnitt 46 und insbesondere die Betätigungsfläche 48 des Hebelbetätigungsabschnitts 46 von den Zähnen 32 des Zahnstangenelements 28 zu beabstanden.

Zusätzlich zu dem Wegerstrecken des Hebelbetätigungsabschnitts 46 und somit der Betätigungsfläche 48 von dem Raum R sind der Hebelbetätigungsabschnitt 46 und die Betätigungsfläche 48 in gewisser Weise gekrümmt oder abgewinkelt oder gewölbt ausgebildet, wobei die Wegkrümmung bzw. Wegwölbung bzw. Abwinkelung insbesondere weg von den Zähnen 32 des Zahnstangenelements 28, insbesondere in distale Richtung (in dem montierten Zustand des Betätigungshebels 44) erfolgt.

In anderen Worten erstreckt sich der Hebelbetätigungsabschnitt 46 des Betätigungshebels 44 im Wesentlichen in einer Richtung von den Instrumentenbranchen 4, 6 und dem Zahnstangenelement 28 weg, welche näherungsweise parallel zu einer Schwenkachse des Scharniergelenks 16 ist, bzw. welche näherungsweise parallel zu einer Schwenkachse des Betätigungshebels 44 ist, wobei der Hebelbetätigungsabschnitt 46 zumindest im Bereich seiner Betätigungsfläche 48 zusätzlich von den Zähnen 32 des Zahnstangenelements 28 gekrümmt bzw. gewölbt ausgebildet ist, 3, um eine ergonomische Betätigungsfläche 48 bereitzustellen und die Betätigungsfläche 48 von den Zähnen 32 des Zahnstangenelements 28 weiter zu beabstanden.

Wie insbesondere aus den Fign. 5, 6 und 7 hervorgeht, ist die Betätigungsfläche 48 des Hebelbetätigungsabschnitts 46 gerippt/ gewellt/ gerillt ausgebildet. Die Betätigungsfläche 48 weist eine Fläche von 0,5 bis 1,5 cm² auf und ist somit geeignet an die Fingerspitze eines Daumens angepasst. Der Hebellagerabschnitt 50 des Betätigungshebels 44 weist zapfenförmige Vorsprünge 54, 56 auf, welche vorgesehen und eingerichtet sind, um in an der zweiten Instrumentenbranche 6 vorgesehene Ausnehmungen eingebracht zu werden, für eine schwenkbare Lagerung des Betätigungshebels 44 an der zweiten Instrumentenbranche 6. Der Hebeleingriffsabschnitt 52 des Betätigungshebels 44 ist ballenartig/ kugelartig ausgebildet, insbesondere gerundet/ abgerundet, speziell in einem Bereich bzw. Abschnitt, welcher vorgesehen ist, in Kontakt bzw. Eingriff mit dem Zahnstangenelement 28, insbesondere den Zähnen 32 des Zahnstangenelements 28, zu kommen.

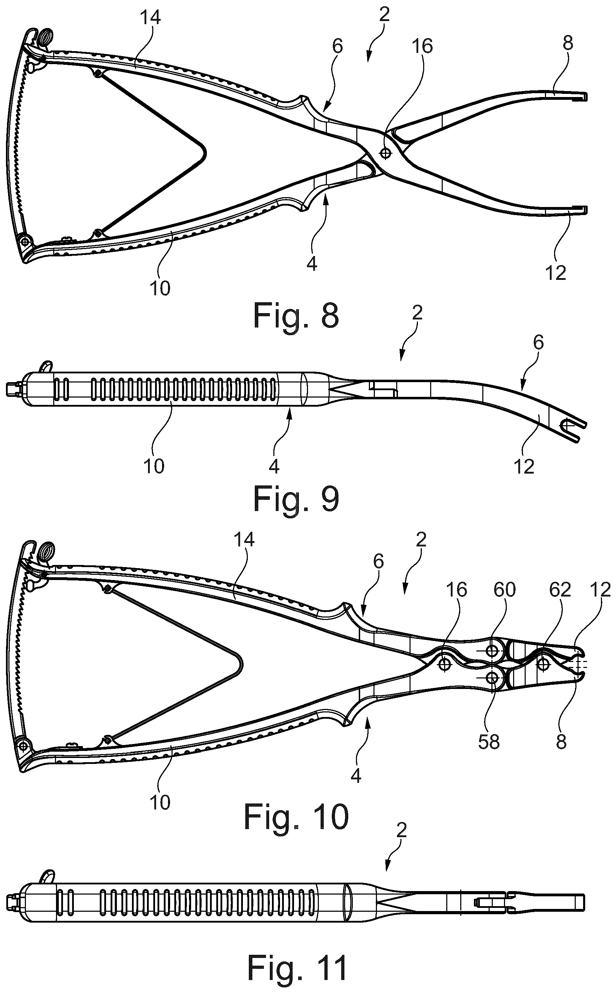

Fig. 8 zeigt eine Vorderansicht und Fig. 9 zeigt eine Unteransicht eines offenbarungsgemäßen medizinischen bzw. chirurgischen Instruments 2 in Form einer Kompressionszange, mittels welcher ein Kompressionsmanöver an Schraubenköpfen von benachbarten Pedikelschrauben während eines Operationsvorgangs durchgeführt werden kann, um die Schraubenköpfe zu einer gewünschten Position zu bewegen und somit zu repositionieren. Es werden lediglich die Unterschiede zu der voranstehend beschriebenen Distraktionszange beschrieben. Insbesondere sind gemäß dieser Ausführungsform die erste Instrumentenbranche 4 und die zweite Instrumentenbranche 6 an dem Scharniergelenk 16 relativ zueinander verschwenkbar gekoppelt, und zwar derart, dass ein Aufeinander-Zu-Bewegen der proximalen Instrumentenbetätigungsabschnitte 10, 14 ein Aufeinander-Zu-Bewegen der distalen Eingriffsabschnitte 8, 12, welche gemäß der Ausführungsform der Fig. 8 auch als Kompressionsabschnitte bezeichnet werden können, bewirkt, wodurch das angesprochene Kompressionsmanöver durchführbar ist. Dies wird insbesondere dadurch erreicht, dass sich die beiden Instrumentenbranchen 4, 6 an dem Scharniergelenk 16 überkreuzen.

Fig. 10 zeigt eine Vorderansicht und Fig. 11 zeigt eine Unteransicht eines offenbarungsgemäßen medizinischen bzw. chirurgischen Instruments 2 in Form einer Stabhaltezange. Die Stabhaltezange ist geeignet bzw. vorgesehen, einen Stab zu halten, welcher auf Pedikelschrauben zu positionieren ist. Es werden lediglich die Unterschiede zu den voranstehend beschriebenen Distraktion- und Kompressionszangen beschrieben. Insbesondere sind bei der Stabhaltezange distal neben dem Scharniergelenk 16 drei weitere Gelenke 58, 60, 62 vorgesehen, welche es ermöglichen, dass bei einem Aufeinander-Zu-Bewegen der proximalen Instrumentenbetätigungsabschnitte 10, 14 ein Stab von den distalen Eingriffsabschnitten 8, 12 auf geeignete Weise gehalten werden kann. Auch wenn in der Ausführungsform der Fig. 10 und Fig. 11 mehrere Gelenke 16, 58, 60, 62 vorgesehen sind, sind die distalen Eingriffsabschnitte 8 bzw. 12 als Teil der entsprechenden Instrumentenbranchen 4 bzw. 6 zu verstehen.

### Bezugszeichenliste

- 2: medizinisches bzw. chirurgisches Instrument
- 4: erste Instrumentenbranche
- 6: zweite Instrumentenbranche
- 8: erster distaler Eingriffsabschnitt
- 10: erster proximaler Betätigungsabschnitt
- 12: zweiter distaler Eingriffsabschnitt
- 14: zweiter proximaler Betätigungsabschnitt
- 16: Scharniergelenk
- 18: Blattfederanordnung
- 20: erste Branchengrifffläche
- 22: zweite Branchengrifffläche
- 24: erster (distaler) Vorsprung
- 26: zweiter (distaler) Vorsprung
- 28: Zahnstangenelement
- 30: Gelenk
- 32: Zahn
- 34: Zahneingriffsfläche
- 36: Nichteingriffsfläche
- 38: erste Seitenfläche
- 40: zweite Seitenfläche
- 42: Rastnase
- 44: Betätigungshebel
- 46: Hebelbetätigungsabschnitt
- 48: Betätigungsfläche
- 50: Hebellagerabschnitt
- 52: Hebeleingriffsabschnitt
- 54: erster zapfenförmiger Vorsprung
- 56: zweiter zapfenförmiger Vorsprung
- 58: Gelenk
- 60: Gelenk
- 62: Gelenk

## Patentansprüche

1. Medizinisches bzw. chirurgisches Instrument (2) mit:
einer ersten Instrumentenbranche (4), die einen ersten proximalen Instrumentenbetätigungsabschnitt (10) aufweist;
einer zweiten Instrumentenbranche (6), die einen zweiten proximalen Instrumentenbetätigungsabschnitt (14) aufweist;
einem an der ersten Instrumentenbranche (4) angebundenen Zahnstangenelement (28);
einer an der zweiten Instrumentenbranche (6) vorgesehenen Rastnase (42), mit welcher das Zahnstangenelement (28) in verrastenden Eingriff bringbar ist;
einem ergonomisch betätigbaren und eine Einhand-Betätigung ermöglichenden Betätigungshebel (44), welcher vorgesehen und eingerichtet ist, bei Betätigung desselben das Zahnstangenelement (28) aus dem verrastenden Eingriff mit der Rastnase (42) zu bringen, wobei
der Betätigungshebel (44) einen Hebelbetätigungsabschnitt (46) mit einer Betätigungsfläche (48) aufweist, welche vorgesehen ist, von einem Anwender betätigt zu werden;
**dadurch gekennzeichnet, dass**
der Hebelbetätigungsabschnitt (46) und/oder dessen Betätigungsfläche (48) von Zähnen (32) des Zahnstangenelements (28) in distale Richtung weggekrümmt oder weggewölbt ist, zur Bereitstellung einer ergonomischen Betätigungsfläche (48) und zur Beabstandung der Betätigungsfläche (48) von den Zähnen (32) des Zahnstangenelements (28).

2. Medizinisches bzw. chirurgisches Instrument (2) nach Anspruch 1, **dadurch gekennzeichnet, dass** das Zahnstangenelement (28) an einem proximalen Ende des ersten proximalen Instrumentenbetätigungsabschnitts (10) schwenkbar gelagert ist und eine Vielzahl von Zähnen (32) aufweist, welche vorgesehen und eingerichtet sind, mit der an einem proximalen Ende des zweiten proximalen Instrumentenbetätigungsabschnitts (14) vorgesehenen Rastnase (42) in verrastenden Eingriff gebracht zu werden.

3. Medizinisches bzw. chirurgisches Instrument (2) nach Anspruch 1 oder 2, **dadurch gekennzeichnet, dass** der Betätigungshebel (44) einen Hebellagerabschnitt (50) aufweist, über welchen der Betätigungshebel (44) schwenkbar an dem zweiten proximalen Instrumentenbetätigungsabschnitt (14) gelagert ist.

4. Medizinisches bzw. chirurgisches Instrument (2) nach einem der vorhergehenden Ansprüche, **dadurch gekennzeichnet, dass** der Betätigungshebel (44) einen Hebeleingriffsabschnitt (52) aufweist, welcher vorgesehen und eingerichtet ist, mit dem Zahnstangenelement (28) in Eingriff gebracht zu werden, um das Zahnstangenelement (28) aus der Rastnase (42) zu hebeln.

5. Medizinisches bzw. chirurgisches Instrument (2) nach einem der vorhergehenden Ansprüche, **dadurch gekennzeichnet, dass** die proximalen Instrumentenbetätigungsabschnitte (10, 14) einen Raum (R) aufspannen bzw. definieren, in welchem sich das Zahnstangenelement (28) vollständig befindet, wobei die Betätigungsfläche (48) des Hebelbetätigungsabschnitts (46) zumindest teilweise außerhalb des Raums (R) angeordnet ist, insbesondere in Bezug auf eine den Raum (R) seitlich begrenzenden Randebene (RE).

6. Medizinisches bzw. chirurgisches Instrument (2) nach einem der vorhergehenden Ansprüche, **dadurch gekennzeichnet, dass** sich der Hebelbetätigungsabschnitt (46) in einer Richtung von den beiden Instrumentenbrachen (4, 6) und dem Zahnstangenelement (28) wegerstreckt, insbesondere zumindest eine Richtungskomponente aufweist, welche parallel zu einer Schwenkachse des Betätigungshebels (44) ist.

7. Medizinisches bzw. chirurgisches Instrument (2) nach einem der vorhergehenden Ansprüche, **dadurch gekennzeichnet, dass** das Zahnstangenelement (28) eine Zähne (32) aufweisende Zahneingriffsfläche (34), eine der Zahneingriffsfläche (34) gegenüberliegenden Nichteingriffsfläche (36), sowie erste und zweite Seitenflächen (38, 40), welche die Zahneingriffsfläche (34) und die Nichteingriffsfläche (36) verbinden, aufweist, wobei die Betätigungsfläche (48) des Hebelbetätigungsabschnitts (46) vollständig von der ersten Seitenfläche (38) in einer Richtung weg von der Zahneingriffsfläche (34) beabstandet ist und sich der Hebelbetätigungsabschnitt (46) in Bezug auf die erste Seitenfläche (38) von dieser wegerstreckt, um den Hebelbetätigungsabschnitt (46) und die Betätigungsfläche (48) des Hebelbetätigungsabschnitts (46) von den Zähnen (32) des Zahnstangenelements (28) zu beabstanden.

8. Medizinisches bzw. chirurgisches Instrument (2) nach einem der vorhergehenden Ansprüche, **dadurch gekennzeichnet, dass** der Hebelbetätigungsabschnitt (46) ein gekrümmter oder gewölbter oder abgewinkelter Abschnitt ist und/oder dass die Betätigungsfläche (48) des Hebelbetätigungsabschnitts (46) eine gekrümmte oder gewölbte oder abgewinkelte Fläche ist.

9. Medizinisches bzw. chirurgisches Instrument (2) n nach einem der vorhergehenden Ansprüche, **dadurch gekennzeichnet, dass** die Betätigungsfläche (48) des Hebelbetätigungsabschnitts (46) eine gekrümmte oder gewölbte Fläche und somit keine ebene Fläche ist.

10. Medizinisches bzw. chirurgisches Instrument (2) nach einem der vorhergehenden Ansprüche, **dadurch gekennzeichnet, dass** die Betätigungsfläche (48) des Hebelbetätigungsabschnitts (46) in ihrer Größe an eine Fingerspitze eines Daumens angepasst ist, insbesondere eine Fläche von 0,5 bis 3 cm², bevorzugt eine Fläche von 0,5 bis 1,5 cm², aufweist.

11. Medizinisches bzw. chirurgisches Instrument (2) nach einem der vorhergehenden Ansprüche, **dadurch gekennzeichnet, dass** der erste proximale Instrumentenbetätigungsabschnitt (10) eine erste Branchengrifffläche (20) aufweist und der zweite proximale Instrumentenbetätigungsabschnitt (14) eine zweite Branchengrifffläche (22) aufweist, wobei die proximalen Instrumentenbetätigungsabschnitte (10, 14) vorgesehen sind, um von einer Hand eines Anwenders derart umgriffen zu werden, dass Finger der Hand an der ersten Branchengrifffläche (20) anliegen, und dass ein Handballen oder ein Mittelhandabschnitt der Hand an der zweiten Branchengrifffläche (22) anliegt.

12. Medizinisches bzw. chirurgisches Instrument (2) nach einem der vorhergehenden Ansprüche, **dadurch gekennzeichnet, dass** die erste Instrumentenbranche (4) und die zweite Instrumentenbrache (6) relativ zueinander verschwenkbar gekoppelt sind.

13. Medizinisches bzw. chirurgisches Instrument (2) nach einem der vorhergehenden Ansprüche, **dadurch gekennzeichnet, dass** das medizinisches bzw. chirurgische Instrument (2) eine Zange, insbesondere eine Stabhaltezange oder eine Kompressionszange oder eine Distraktionszange ist.

## Claims

1. A medical or surgical instrument (2) comprising:
a first instrument branch (4) having a first proximal instrument actuation portion (10);
a second instrument branch (6) comprising a second proximal instrument actuation portion (14);
a rack element (28) connected to the first instrument branch (4);
a snap-in nose (42) provided on the second instrument branch (6) and with which the rack element (28) can be brought into locking engagement;
an ergonomically actuable actuation lever (44) enabling a one-handed actuation, which is provided and adapted to bring the rack element (28) out of locking engagement with the snap-in nose (42) upon actuation thereof, wherein
the actuation lever (44) comprises a lever actuation portion (46) with an actuation surface (48) which is provided to be actuated by a user;
**characterized in that**
the lever actuation portion (46) and/or the actuation surface (48) thereof is curved away or arched away from teeth (32) of the rack element (28) in the distal direction, for providing an ergonomic actuation surface (48) and for spacing the actuation surface (48) from the teeth (32) of the rack element (28).

2. The medical or surgical instrument (2) according to claim 1, **characterized in that** the rack element (28) is pivotably mounted at a proximal end of the first proximal instrument actuation portion (10) and comprises a plurality of teeth (32) which are provided and adapted to be brought into locking engagement with the snap-in nose (42) provided at a proximal end of the second proximal instrument actuation portion (14).

3. The medical or surgical instrument (2) according to claim 1 or 2, **characterized in that** the actuation lever (44) comprises a lever bearing portion (50) via which the actuation lever (44) is pivotably mounted at the second proximal instrument actuation portion (14).

4. The medical or surgical instrument (2) according to one of the preceding claims, **characterized in that** the actuation lever (44) comprises a lever engagement portion (52) which is provided and adapted to be brought into engagement with the rack element (28) in order to lever the rack element (28) out of the snap-in nose (42).

5. The medical or surgical instrument (2) according to one of the preceding claims, **characterized in that** the proximal instrument actuation portions (10, 14) span or define a space (R) in which the rack element (28) is completely located, wherein the actuation surface (48) of the lever actuation portion (46) is arranged at least partially outside the space (R), in particular with respect to an edge plane (RE) laterally delimiting the space (R).

6. The medical or surgical instrument (2) according to one of the preceding claims, **characterized in that** the lever actuation portion (46) extends away from the two instrument branches (4, 6) and the rack element (28) in one direction, in particular comprises at least one directional component which is parallel to a pivot axis of the actuation lever (44).

7. The medical or surgical instrument (2) according to one of the preceding claims, **characterized in that** the rack element (28) comprises a tooth engagement surface (34) comprising teeth (32), a non-engagement surface (36) opposite the tooth engagement surface (34), as well as first and second side surfaces (38, 40) which connect the tooth engagement surface (34) and the non-engagement surface (36), wherein the actuation surface (48) of the lever actuation portion (46) is completely spaced from the first side surface (38) in a direction away from the tooth engagement surface (34), and the lever actuation portion (46) extends away from the first side surface (38) with respect thereto, in order to space the lever actuation portion (46) and the actuation surface (48) of the lever actuation portion (46) from the teeth (32) of the rack element (28).

8. The medical or surgical instrument (2) according to one of the preceding claims, **characterized in that** the lever actuation portion (46) is a curved or arched or angled portion and/or that the actuation surface (48) of the lever actuation portion (46) is a curved or arched or angled surface.

9. The medical or surgical instrument (2) according to one of the preceding claims, **characterized in that** the actuation surface (48) of the lever actuation portion (46) is a curved or arched surface and thus not a planar surface.

10. The medical or surgical instrument (2) according to one of the preceding claims, **characterized in that** the actuation surface (48) of the lever actuation portion (46) is adapted in its size to a fingertip of a thumb, in particular has an area of 0.5 to 3 cm², preferably an area of 0.5 to 1.5 cm².

11. The medical or surgical instrument (2) according to one of the preceding claims, **characterized in that** the first proximal instrument actuation portion (10) has a first branch grip surface (20) and the second proximal instrument actuation portion (14) has a second branch grip surface (22), wherein the proximal instrument actuation portions (10, 14) are provided to be gripped by a hand of a user such that fingers of the hand lie against the first branch grip surface (20), and that a ball of the hand or a middle-hand portion of the hand lies against the second branch grip surface (22).

12. The medical or surgical instrument (2) according to one of the preceding claims, **characterized in that** the first instrument branch (4) and the second instrument branch (6) are coupled pivotably relative to each other.

13. The medical or surgical instrument (2) according to one of the preceding claims, **characterized in that** the medical or surgical instrument (2) is forceps, in particular rod holding forceps or compression forceps or distraction forceps.

## Revendications

1. Instrument médical ou chirurgical (2) avec :
une première branche d'instrument (4) qui présente une première section d'actionnement d'instrument (10) proximale ;
une seconde branche d'instrument (6) qui présente une seconde section d'actionnement d'instrument (14) proximale ;
un élément de crémaillère (28) liée à la première branche d'instrument (4) ;
un nez d'encliquetage (42) prévu au niveau de la seconde branche d'instrument (6) avec lequel l'élément de crémaillère (28) peut être amené en prise d'encliquetage ;
un levier d'actionnement (44) permettant un actionnement à une main et actionnable de manière ergonomique qui est prévu et conçu afin d'amener l'élément de crémaillère (28) lors de l'actionnement du levier hors de la prise d'encliquetage avec le nez d'encliquetage (42), dans lequel
le levier d'actionnement (44) présente une section d'actionnement de levier (46) avec une surface d'actionnement (48) qui est prévue afin d'être actionnée par un utilisateur ;
**caractérisé en ce que**
la section d'actionnement de levier (46) et/ou sa surface d'actionnement (48) est courbée ou bombée en s'éloignant des dents (32) de l'élément de crémaillère (28) en direction distale pour fournir une surface d'actionnement (48) ergonomique et pour espacer la surface d'actionnement (48) des dents (32) de l'élément de crémaillère (28).

2. Instrument médical ou chirurgical (2) selon la revendication 1, **caractérisé en ce que** l'élément de crémaillère (28) est logé de manière pivotante au niveau d'une extrémité proximale de la première section d'actionnement d'instrument (10) proximale et présente une pluralité de dents (32) qui sont prévues et conçues afin d'être amenées en prise d'encliquetage avec le nez d'encliquetage (42) prévu au niveau d'une extrémité proximale de la seconde section d'actionnement d'instrument (14) proximale.

3. Instrument médical ou chirurgical (2) selon la revendication 1 ou 2, **caractérisé en ce que** le levier d'actionnement (44) présente une section de palier de levier (50) par le biais de laquelle le levier d'actionnement (44) est logé de manière pivotante au niveau de la seconde section d'actionnement d'instrument (14) proximale.

4. Instrument médical ou chirurgical (2) selon l'une quelconque des revendications précédentes, **caractérisé en ce que** le levier d'actionnement (44) présente une section de prise de levier (52) qui est prévue et conçue afin d'être amenée en prise avec l'élément de crémaillère (28) afin de lever l'élément de crémaillère (28) du nez d'encliquetage (42).

5. Instrument médical ou chirurgical (2) selon l'une quelconque des revendications précédentes, **caractérisé en ce que** les sections d'actionnement d'instrument (10, 14) proximales tendent ou définissent un espace (R) dans lequel l'élément de crémaillère (28) se situe complètement, dans lequel la surface d'actionnement (48) de la section d'actionnement de levier (46) est agencée au moins partiellement en dehors de l'espace (R), en particulier par rapport à un plan de bord (RE) délimitant latéralement l'espace (R).

6. Instrument médical ou chirurgical (2) selon l'une quelconque des revendications précédentes, **caractérisé en ce que** la section d'actionnement de levier (46) s'étend dans une direction s'éloignant des deux branches d'instrument (4, 6) et de l'élément de crémaillère (28), en particulier présente au moins un composant de direction qui est parallèle à un axe de pivotement du levier d'actionnement (44).

7. Instrument médical ou chirurgical (2) selon l'une quelconque des revendications précédentes, **caractérisé en ce que** l'élément de crémaillère (28) présente une surface de prise dentée (34) présentant des dents (32), une surface de non-prise (36) opposée à la surface de prise dentée (34), ainsi que des premières et secondes surfaces latérales (38, 40) qui relient la surface de prise dentée (34) et la surface de non-prise de dent (36), dans lequel la surface d'actionnement (48) de la section d'actionnement de levier (46) est complètement espacée de la première surface latérale (38) dans une direction s'éloignant de la surface de prise de dent (34), et la section d'actionnement de levier (46) s'étend par rapport à la première surface latérale (38) en s'éloignant de celle-ci afin d'espacer la section d'actionnement de levier (46) et la surface d'actionnement (48) de la section d'actionnement de levier (46) des dents (32) de l'élément de crémaillère (28).

8. Instrument médical ou chirurgical (2) selon l'une quelconque des revendications précédentes, **caractérisé en ce que** la section d'actionnement de levier (46) est une section courbée ou bombée ou coudée et/ou **en ce que** la surface d'actionnement (48) de la section d'actionnement de levier (46) est une surface courbée ou bombée ou coudée.

9. Instrument médical ou chirurgical (2) selon l'une quelconque des revendications précédentes, **caractérisé en ce que** la surface d'actionnement (48) de la section d'actionnement de levier (46) est une surface courbée ou bombée et n'est ainsi pas une surface plane.

10. Instrument médical ou chirurgical (2) selon l'une quelconque des revendications précédentes, **caractérisé en ce que** la surface d'actionnement (48) de la section d'actionnement de levier (46) est adaptée dans sa grandeur à un bout de doigt d'un pouce, en particulier une surface de 0,5 à 3 cm², de préférence une surface de 0,5 à 1,5 cm².

11. Instrument médical ou chirurgical (2) selon l'une quelconque des revendications précédentes, **caractérisé en ce que** la première section d'actionnement d'instrument (10) proximale présente une première surface de préhension de branche (20) et la seconde section d'actionnement d'instrument (14) proximale présente une seconde surface de préhension de branche (22), dans lequel les sections d'actionnement d'instrument (10, 14) proximales sont prévues afin d'être entourées par une main d'un utilisateur de telle manière que des doigts de la main reposent sur la première surface de préhension de branche (20) et qu'une paume ou une section de métacarpe de la main repose sur la seconde surface de préhension de branche (22).

12. Instrument médical ou chirurgical (2) selon l'une quelconque des revendications précédentes, **caractérisé en ce que** la première branche d'instrument (4) et la seconde branche d'instrument (6) sont couplées l'une par rapport à l'autre de manière pivotante.

13. Instrument médical ou chirurgical (2) selon l'une quelconque des revendications précédentes, **caractérisé en ce que** l'instrument médical ou chirurgical (2) est une pince, en particulier une pince de support de barre ou une pince de compression ou une pince de distraction.
